# EUROPEAN PATENT APPLICATION

(11) **EP 3 454 056 A1**
(43) Date of publication of application: **13.03.2019**
(21) Application number: 16891576.7
(22) Date of filing: 14.09.2016
(51) Int. Cl.: G01N 27/327, G01N 27/416, G01N 33/72

(54) **BIOSENSOR**

(30) Priority: 25.02.2016 JP 2016034671
(71) Applicant: PHC Holdings Corporation, Tokyo 105-8433 (JP)
(72) Inventor: SHIGETOU, Nobuyuki, Ehime 791-0395 (JP); HANEDA, Keigo, Ehime 791-0395 (JP); OOTANI, Shingo, Ehime 791-0395 (JP)
(74) Representative: Vigand, Philippe
(86) International application number: PCT/JP2016/077154
(87) International publication number: WO 2017/145420

(57) **Abstract**

A biosensor includes a base substrate **2** having a main surface; an electrode layer **4** located on the main surface of the base substrate **2** and including a first electrode pair **4a** and a second electrode pair **4b** each including a working electrode and a counter electrode; a spacer substrate **8** located on the electrode layer and having a first opening **8a** and a second opening **8b** formed therein, the first opening **8a** exposing a part of the first electrode pair and the second opening **8b** exposing a part of the second electrode pair, the second opening being independent from the first opening; a first reagent layer **6A** containing a first reagent reactive with a sample, the first reagent layer **6A** being located in the first opening; a second reagent layer **6B** containing a second reagent reactive with the sample, the second reagent layer **6B** being located in the second opening; and a top cover substrate **10** located on the spacer substrate, the top cover substrate **10** including an introduction portion **10c** having at least one introduction opening formed therein, the introduction portion overlapping a part of the first opening in the spacer substrate and a part of the second opening in the spacer substrate.

## Description

### TECHNICAL FIELD

This application relates to a biosensor measuring the concentration of a specific substance contained in a sample.

### BACKGROUND ART

Biosensors have been put into practice in various fields including the medical and clinical inspection and pharmaceutical manufacturing. For example, a biosensor measuring the concentration of various substances in the blood has been put into practice and are now widely used in the field of medical and clinical inspection.

Patent Document 1 discloses a microfluidic sensor usable as an electrochemical blood test strip for blood coagulation measurement or the like. The microfluidic sensor includes a flow path including branches in which fluids to be measured flow by the capillary action and an electrode pair provided in a reaction zone located at ends of the branches of the flow path. Patent Document 1 discloses that coagulation time is important for blood coagulation measurement and that it is important to minimize the factor preventing the flow of the fluids in the reaction zone in order to perform the measurement with high precision and high reproducibility.

### CITATION LIST

### PATENT LITERATURE

Patent Document 1: Japanese Laid-Open Patent Publication No. 2015-108622

### SUMMARY OF INVENTION

### TECHNICAL PROBLEM

It is preferable that the structure of a biosensor is optimized for the properties of a subject to be detected and the measurement method used for the detection. This disclosure provides a biosensor using an electrochemical measurement method capable of measuring two or more types of substances more precisely.

### SOLUTION TO PROBLEM

A biosensor of this disclosure includes a base substrate having a main surface; an electrode layer located on the main surface of the base substrate and including a first electrode pair and a second electrode pair each including a working electrode and a counter electrode; a spacer substrate located on the electrode layer and having a first opening and a second opening formed therein, the first opening exposing a part of the first electrode pair and the second opening exposing a part of the second electrode pair, the second opening being independent from the first opening; a first reagent layer containing a first reagent reactive with a sample, the first reagent layer being located in the first opening; a second reagent layer containing a second reagent reactive with the sample, the second reagent layer being located in the second opening; and a top cover substrate located on the spacer substrate, the top cover substrate including an introduction portion having at least one introduction opening formed therein, the introduction portion overlapping a part of the first opening and a part of the second opening in the spacer substrate.

### ADVANTAGEOUS EFFECTS OF INVENTION

With the biosensor of this disclosure, the introduction opening in the top cover substrate overlaps the first opening and the second opening. Therefore, the sample is introduced into the first opening and the second opening in which the electrodes are provided, with no need to provide a flow path including branches for introducing the sample into reaction chambers. With such a structure, the area size of the first opening and/or the second opening may be enlarged to increase the amount of the sample used for the measurement, and also the length of a border between the working electrode and the counter electrodes can be extended. Thus, the detection sensitivity of the biosensor is improved. Therefore, two or more types of substances may be measured with high precision by an electrochemical measurement method.

### BRIEF DESCRIPTION OF DRAWINGS

FIG. **1(a)** and FIG. **1(b)** are respectively an isometric view and an exploded isometric view of a biosensor in embodiment 1.
FIG. **2(a)** and FIG. **2(b)** are respectively a cross-sectional view and a plan view of the biosensor shown in FIG. **1****;** FIG. **2(c)** is a plan view of the biosensor in the state where a top cover substrate is removed; FIG. **2(d)** is a plan view of the biosensor in the state where the top cover substrate and first and second reagent layers are removed; and FIG. **2(e)** is a plan view of the biosensor in the state where a spacer substrate and components above the spacer substrate are removed.
FIG. **3** is a block diagram showing a structure of a measurement device.
FIG. **4** is an isometric view showing how a sample is dropped to the biosensor.
FIG. **5** is a graph showing the relationship between the concentration of HbAlc and the current value in the case where measurement is performed on HbA1c, the concentration of which is known, by use of the biosensor in embodiment 1.
FIG. **6** is a graph showing the relationship between the concentration of Hb and the current value in the case where measurement is performed on Hb, the concentration of which is known, by use of the biosensor in embodiment 1.
FIG. **7(a)** and FIG. **7(b)** are respectively an isometric view and an exploded isometric view of a biosensor in embodiment 2.
FIG. **8** is an exploded isometric view of a biosensor in embodiment 3.
FIG. **9** is an exploded isometric view of a biosensor in embodiment 4.

### DESCRIPTION OF EMBODIMENTS

The present inventors studied, in detail, the structure of a biosensor capable of measuring two or more types of substances by an electrochemical measurement method with high precision. It is now assumed that, for example, the value of hemoglobin A1c (hereinafter, referred to as "HbA1c") is to be measured electrochemically by a biosensor. The HbA1c value is an inspection value that shows the ratio of hemoglobin bonded with sugar (HbA1c) with respect to hemoglobin in the erythrocytes (referred to as "Hb") . The HbAlc value reflects an average blood sugar level in the past one to two months, and therefore, is less likely to be influenced by the pre-inspection meal than the blood glucose level and thus is usable as a more accurate index for diabetes control. In order to find the HbAlc value, the concentration (amount) of HbAlc and the concentration (amount) of Hb need to be found. The term "Hb" encompasses HbAlc and all the other derivatives. The concentration of HbA1c is relatively low and thus is difficult to be measured especially electrochemically. Therefore, the HbA1c value has been measured mainly by an optical spectroscopic method.

In the case where the concentration of the substance to be measured by a biosensor using an electrochemical method is low, it is conceivable to use a maximum possible amount of sample and to enlarge a detection region of the electrode to increase the detection precision. A conventional biosensor detecting two or more types of substances divides the sample into two or more by use of a microfluidic path including branches and detects the substances in the sample in each of reaction chambers by use of an electrochemical reaction. However, the biosensor of such a structure, in which a microfluidic path including branches is formed, may occasionally not allow formation of a large electrode. In light of such a problem, the present inventors conceived a biosensor having a novel structure. The overview of the biosensor in this disclosure is as follows.

### [Item 1]

A biosensor, comprising:
a base substrate having a main surface;
an electrode layer located on the main surface of the base substrate and including a first electrode pair and a second electrode pair each including a working electrode and a counter electrode;
a spacer substrate located on the electrode layer and having a first opening and a second opening formed therein, the first opening exposing a part of the first electrode pair and the second opening exposing a part of the second electrode pair, the second opening being independent from the first opening;
a first reagent layer containing a first reagent reactive with a sample, the first reagent layer being located in the first opening;
a second reagent layer containing a second reagent reactive with the sample, the second reagent layer being located in the second opening; and
a top cover substrate located on the spacer substrate, the top cover substrate including an introduction portion having at least one introduction opening formed therein, the introduction portion overlapping a part of the first opening in the spacer substrate and a part of the second opening in the spacer substrate.

With this structure, the introduction opening in the top cover substrate overlaps the first opening and the second opening. Therefore, the sample is introduced into the first opening and the second opening in which the electrodes are provided, with no need to provide a flow path including branches for introducing the sample into reaction chambers. With such a structure, the area size of the first opening and/or the second opening may be enlarged to increase the amount of the sample used for the measurement, and also the length of a border between the working electrode and the counter electrodes can be extended. Thus, the detection sensitivity of the biosensor is improved. Since the sample is introduced from the introduction portion, the sample is introduced into the first opening and the second opening provided in the spacer substrate at the same time in parallel to each other. Therefore, the time required to fill the first opening and the second opening with the sample to start the measurement is shortened. Since the first opening and the second opening are independent from each other, the size of each thereof may be set arbitrarily. The amounts of the sample to be used for the first opening and the second opening are adjusted independently. Therefore, such amounts of the sample may be made different in accordance with the concentrations, to be detected, of the substances in the sample, and also in accordance with the detection sensitivity on each of the substances. The measurement is performed with appropriate sensitivity.

The top cover substrate includes the introduction portion. Therefore, the amount of the sample to be dropped may be increased to fill the first opening and the second opening with the sample within a short time. This shortens the measurement time. Even if the area size of each of the first electrode pair and the second electrode pair for the measurement is enlarged, the sample of a sufficient amount is put into contact with the electrode pairs within a short time. This improves the detection sensitivity.

### [Item 2]

The biosensor according to item 1, wherein in the spacer substrate, the first opening has an area size larger than an area size of the second opening.

With this structure, the first opening is large. Therefore, a larger amount of sample may be used for the measurement, and thus the detection precision of the substance to be detected by use of the first substance is improved.

### [Item 3]

The biosensor according to item 1 or 2, wherein in the electrode layer, a length of a border between the working electrode and the counter electrode adjacent thereto of the first electrode pair exposed to the first opening is longer than a length of a border between the working electrode and the counter electrode adjacent thereto of the second electrode pair exposed to the second opening.

With this structure, the length of the border between the working electrode and the counter electrode of the first electrode pair may be extended. This improves the detection sensitivity.

### [Item 4]

The biosensor according to item 1, further comprising a first discharge opening and a second discharge opening respectively in communication with the first opening and the second opening in the spacer substrate.

With this structure, the first discharge opening and the second discharge opening are provided. This allows the sample to be introduced into the first opening and the second opening with certainty by use of the capillary force.

### [Item 5]

The biosensor according to item 4, wherein the first discharge opening and the second discharge opening are provided in the spacer substrate, each have an opening at a side surface of the spacer substrate, and are respectively connected with the first opening and the second opening.

With this structure, the top cover substrate merely has the introduction opening formed in a main surface thereof. Therefore, it is less possible that any foreign object enters the first discharge opening or the second discharge opening or that the operator considers, by mistake, the first discharge opening or the second discharge opening as an introduction opening and introduces the sample through the first discharge opening or the second discharge opening.

### [Item 6]

The biosensor according to item 4, wherein the first discharge opening and the second discharge opening are provided in the top cover substrate and respectively overlap another part of the first opening in the spacer substrate and another part of the second opening in the spacer substrate.

### [Item 7]

The biosensor according to any one of items 1 through 6, wherein the introduction portion of the top cover substrate has one introduction opening formed therein, and the introduction opening overlaps the part of the first opening in the spacer substrate and the part of the second opening in the spacer substrate.

With this structure, the sample may be introduced from the introduction opening into the first opening and the second opening at the same time. This simplifies the operation for the measurement.

### [Item 8]

The biosensor according to any one of items 1 through 6, wherein the introduction portion of the top cover substrate has two introduction openings formed therein, one of the introduction openings overlaps the part of the first opening in the spacer substrate, and the other of the introduction openings overlaps the part of the second opening in the spacer substrate.

With this structure, the introduction openings are respectively provided for the first opening and the second opening in the spacer substrate. Therefore, the capillary forces used to introduce the sample into the first opening and the second opening are more easily adjustable independently. Even in the case where the amounts of the sample to be introduced into the first opening and the second opening are increased because of the difference in the detection sensitivity, the time required for the introduction may be made the same, and the sample is allowed to be introduced into the entirety of the first opening and the second opening with certainty. This shortens the measurement time and realizes the measurement with high reproducibility.

### [Item 9]

The biosensor according to item 1, wherein:
the base substrate, the spacer substrate and the top cover substrate each have a strip shape that is longer in a first direction than in a second direction perpendicular to the first direction;
the first opening and the second opening in the spacer substrate are located adjacent to each other in the first direction; and
the introduction portion of the top cover substrate has one introduction opening formed therein, and the introduction opening overlaps parts of the first opening and the second opening that are adjacent to each other.

### [Item 10]

The biosensor according to item 9, wherein:
the top cover substrate further has a first discharge opening and a second discharge opening formed therein;
the first discharge opening and the introduction opening in the introduction portion respectively overlap two ends in the first direction of the first opening in the spacer substrate; and
the second discharge opening and the introduction opening in the introduction portion respectively overlap two ends in the first direction of the second opening in the spacer substrate.

### [Item 11]

The biosensor according to any one of items 1 through 10, wherein:
the sample contains hemoglobin separated from erythrocytes;
the hemoglobin contains hemoglobin A1c;
the first reagent is specifically reactive with the hemoglobin A1c or a substance derived from the hemoglobin A1c; and
the second reagent is reactive with the hemoglobin.

Hereinafter, embodiments of a biosensor in this disclosure will be described in detail with reference to the drawings. The biosensor of this disclosure electrochemically detects and/or quantifies two or more different substances contained in a sample. In each of the embodiments of this disclosure, one embodiment of a biosensor detecting a first substance and a second substance contained in the sample will be described. Especially in the case where the detection sensitivities on two or more substances contained in the sample are different, a substance with relatively low detection sensitivity may be detected with improved sensitivity by the biosensor of this disclosure. In the following embodiments, the first substance and the second substance are respectively HbAlc and Hb obtained by a preprocess performed on the blood, and the biosensor electrochemically measures the concentrations of the first substance and the second substance. The biosensor of this disclosure is preferably usable to detect any other appropriate substance. The following embodiments are merely illustrative, and the present invention is not limited to any of the following embodiments. In the following embodiments, components are represented by reference signs in the drawings, and substantially the same descriptions may be omitted or components that are not referred to in the description may not be represented by any reference sign, for easier understanding and or avoidance of unnecessary repetitions.

### (EMBODIMENT 1)

### <Structure of the biosensor>

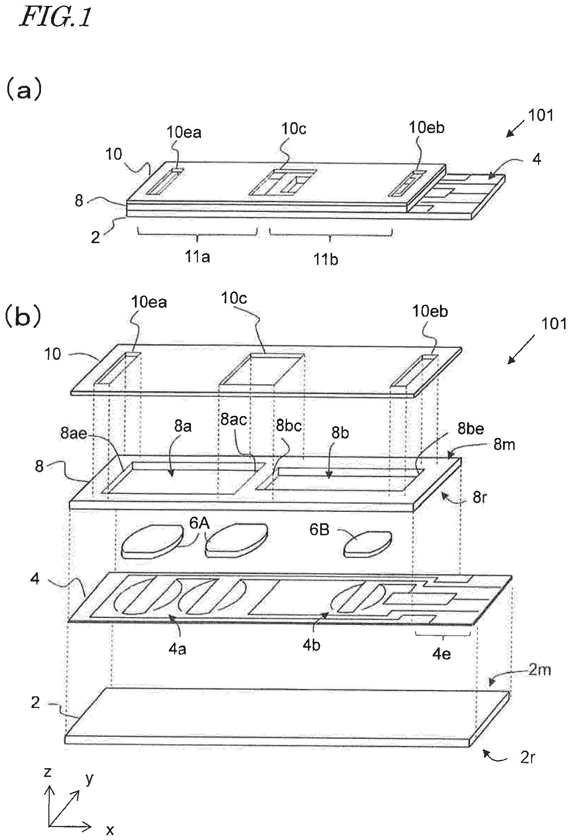

A biosensor in embodiment 1 in this disclosure will be described. FIG. **1(a)** and FIG. **1(b)** are respectively an isometric view and an exploded isometric view of a biosensor **101** in this embodiment. The biosensor **101** includes a base substrate **2,** an electrode layer **4,** first and second reagent layers **6A** and **6B,** a spacer substrate **8,** and a top cover substrate **10.** As shown in FIG. **1(b)****,** the base substrate **2,** the spacer substrate **8** and the top cover substrate **8** each have a strip shape and are each longer in a longitudinal direction thereof, which is along an **x** direction, than in a width direction, which is along a **y** direction.

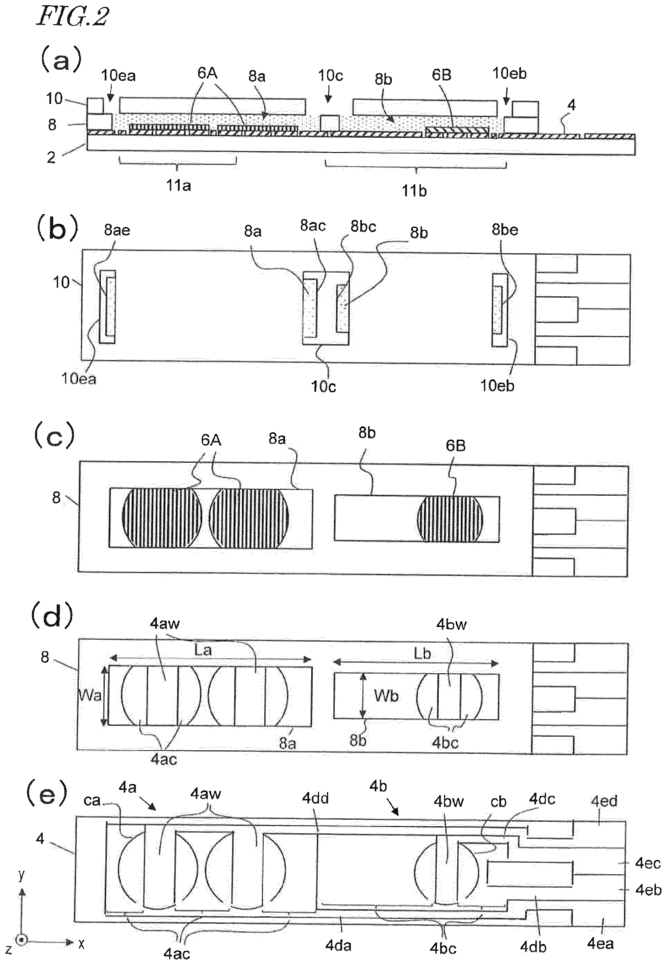

FIG. **2(a)** and FIG. **2(b)** are respectively a cross-sectional view and a plan view of the biosensor **101.** FIG. **2(c),** FIG. **2(d)** and FIG. **2(e)** are respectively a plan view of the biosensor **101** in the state where the top cover substrate **10** is removed, a plan view of the biosensor **101** in the state where the top cover substrate **10** and the first and second reagent layers **6A** and **6B** are removed, and a plan view of the biosensor **101** in the state where the spacer substrate **8** and components above the spacer substrate **8** are removed. With reference to these figures, a structure of the biosensor **101** will be described in detail.

The base substrate **2** supports all the other components of the biosensor **101.** The base substrate **2** has a main surface **2m** and a main surface **2r,** and has a strip shape as described above. A "main surface" of a substrate is a main large surface by which the substrate supports components.

The electrode layer **4** is located on the main surface **2m** of the base substrate **2.** The electrode layer **4** includes at least a first electrode pair **4a** and a second electrode pair **4b.** The first electrode pair **4a** and the second electrode pair **4b** are used to detect a first substance and a second substance, which are different substances contained in a sample. In this embodiment, the first substance is HbA1c, and the second substance is Hb.

As shown in FIG. **2(e)****,** the first electrode pair **4a** includes working electrodes **4aw** and counter electrodes **4ac.** In this embodiment, the first electrode pair **4a** includes two working electrodes **4aw** and three counter electrodes **4ac.** The two working electrodes **4aw** are connected to a terminal **4ed** via a line **4dd.** The three counter electrodes **4ac** are connected to a terminal **4ea** via a line **4da.** In the **x** direction, which is the longitudinal direction of the base substrate **2,** the working electrodes **4aw** and the counter electrodes **4ac** are located alternately.

Similarly, the second electrode pair **4b** includes a working electrode **4bw** and counter electrodes **4bc.** In this embodiment, the second electrode pair **4b** includes one working electrode **4bw** and two counter electrodes **4bc.** The working electrode **4bw** is connected to a terminal **4ec** via a line **4dc.** The two counter electrodes **4bc** are connected to a terminal **4eb** via a line **4db.** In the longitudinal direction of the base substrate **2,** the working electrode **4bw** and the counter electrodes **4bc** are located alternately. A pair of counter electrodes **4ac** having each of the working electrodes **4aw** therebetween each have an arcked cut **ca.** Similarly, the pair of counter electrodes **4bc** having the working electrode **4bw** therebetween each have an arcked cut **cb.**

In this embodiment, the above-described structure of the electrodes in the electrode layer **4** is provided by patterning a metal film covering substantially the entirety of the main surface **2m** of the base substrate **2.**

The spacer substrate **8** is located on the electrode layer **4,** and has a first opening **8a** and a second opening **8b** formed therein. The spacer substrate **8** is shorter than the base substrate **2** in the longitudinal direction. Therefore, the terminals **4ea** through **4ed** are not covered by the spacer substrate **8** and are exposed.

The first opening **8a** and the second opening **8b** are through-holes reaching two main surfaces **8m** and **8r** of the spacer substrate **8.** The first opening **8a** and the second opening **8b** are independent spaces from each other and are not in communication with each other. The spacer substrate **8** is provided between the base substrate **2** and the top cover substrate **10,** and thus the first opening **8a** and the second opening **8b** act as reaction chamber holding a sample and detecting an electric change of the sample caused by reactions of the sample with the first reaction layers **6A** and **6B.**

The first opening **8a** and the second opening **8b** each have a rectangular shape having a longitudinal direction in the longitudinal direction of the spacer substrate **8,** and are arranged in the longitudinal direction of the spacer substrate **8.** Therefore, one end **8ac** of the first opening **8a** is close to one end **8bc** of the second opening **8b** at the center or in the vicinity thereof of the spacer substrate **8.** The other end **8ae** of the first opening **8a** and the other end **8be** of the second opening **8b** are respectively close to two ends, in the longitudinal direction, of the spacer substrate **8.**

In this embodiment, the first substance in the sample held in the first opening **8a** is detected with high sensitivity. Namely, the reaction chamber provided to detect the first substance has a larger capacity than the capacity of the reaction chamber provided to detect the second substance. Therefore, it is preferable that the first opening **8a** has an area size larger than the area size of the second opening **8b.** In this embodiment, as shown in FIG. **2(d)****,** where the length of the first opening **8a** in the **x** direction (longitudinal direction) is **La,** the length of the second opening **8b** in the **x** direction (longitudinal direction) is **Lb,** the width of the first opening **8a** in the **y** direction (direction perpendicular to the longitudinal direction) is **Wa,** and the width of the second opening **8b** in the **y** direction (direction perpendicular to the longitudinal direction) is **Wb,** La > Lb and Wa > Wb. With such an arrangement, the amount of the sample that can be held in the first opening **8a** may be made larger than the amount of the sample that can be held in the second opening **8b.**

The sizes of the first opening **8a** and the second opening **8b** may be determined in accordance with the concentrations of the first substance and the second substance in the sample and the required amount of the sample determined in consideration of, for example, the detection sensitivity by the detection method to be used. In the case where the sample is introduced into the first opening **8a** and the second opening **8b** by use of the capillary force (capillary action), the spacer substrate **8** has a thickness (**z** direction) with which the capillary force may act on the sample.

Since the spacer substrate **8** is located on the electrode layer **4,** a part of the first electrode pair **4a** and a part of the second electrode pair **4b** are respectively exposed to the first opening **8a** and the second opening **8b.** Electric or electrochemical changes of the sample held in the first opening **8a** and the second opening **8b** may be respectively detected by the first electrode pair **4a** and the second electrode pair **4b.** In the first opening **8a,** a total length of borders between the working electrodes **4aw** and the counter electrodes **4ac** of the first electrode pair **4a** is 4 × Wa. In the second opening **8b,** a total length of borders between the working electrode **4bw** and the counter electrodes **4bc** of the first electrode pair **4b** is 2 × Wb. Namely, the total length of the borders between the working electrode (s) and the counter electrodes is longer in the first electrode pair **4a** than in the second electrode pair **4b.** The first electrode pair **4a** has higher detection sensitivity.

The first reagent layer **6A** and the second reagent layer **6B** respectively contain a first reagent and a second reagent. The first reagent and the second reagent respectively react with the first substance and the second substance in the sample, or with substances derived from the first substance and the second substance by preprocessing, to cause an electrically detectable change to the sample. In this embodiment, the first reagent is fructosyl peptide oxidase, which is a detection enzyme specifically reactive with fructosyl valyl histidine, which is generated by a preprocess performed on HbA1c, and is immobilized to the first reagent layer. The second reagent is potassium ferricyanide, which is a mediator reactive with any type of Hb encompassing HbA1c, and is immobilized to the second reagent layer.

The first reagent layer **6A** and the second reagent layer **6B** are respectively located in the first opening **8a** and the second opening **8b** in the spacer substrate **8.** More specifically, the first reagent layer **6A** is located above an area in the vicinity of the borders between the working electrodes **4aw** and the counter electrodes **4ac** of the two first electrode pairs **4a,** and the second reagent layer **6B** is located above an area in the vicinity of the borders between the working electrode **4bw** and the counter electrodes **4bc** of the second electrode pair **4b.** In this embodiment, in the case where the materials of the first reagent layer **6A** and the second reagent layer **6B** are dropped from above the electrode layer **4,** regions in which the materials expand are defined by the arcked cuts **ca** and **cb** formed in the counter electrodes. Therefore, the amount of the first reagent layer **6A** located in the vicinity of the borders between the working electrodes and the counter electrodes, and the amount of the second reagent layer **6B** located in the vicinity of the borders between the working electrode and the counter electrodes, are made the same as each other. As a result, the level of the electric current is suppressed from being varied between biosensor cells at the time of detection of the first substance and the second substance.

The top cover substrate **10** is located on the spacer substrate **8.** The top cover substrate **10** includes an introduction portion having at least one introduction opening formed therein. In this embodiment, the introduction portion has one introduction opening **10c** formed therein. As well shown in FIG. **2(a)** and FIG. **2(b)****,** the introduction opening **10c** overlaps a part of the first opening **8a** and a part of the second opening **8b.** More specifically, the one end **8ac,** in the longitudinal direction, of the first opening **8a** and the one end **8bc,** in the longitudinal direction, of the second opening **8b** are located in the introduction opening **10c.** Therefore, the introduction opening **10c** is in communication with the first opening **8a** and the second opening **8b.** When the sample is dropped to the introduction opening **10c,** the sample flows into the first opening **8a** and the second opening **8b.**

In this embodiment, the top cover substrate **10** further has a first discharge opening **10ea** and a second discharge opening **10eb** formed therein. The first discharge opening **10ea** and the second discharge opening **10eb** respectively overlap a part of the first opening **8a** and a part of the second opening **8b.** The other end **8ae** of the first opening **8a** and the other end **8be** of the second opening **8b** are respectively located in the first discharge opening **10ea** and the second discharge opening **10eb.** Since the first discharge opening **10ea** and the second discharge opening **10eb** are provided, the following occurs. In the case where the biosensor **101** is configured such that the capillary force acts on the first opening **8a** and the second opening **8b,** gas present in the first opening **8a** and the second opening **8b** is discharged from the first discharge opening **10ea** and the second discharge opening **10eb** as the sample is pulled into the first opening **8a** and the second opening **8b.** In this manner, the sample is introduced into the first opening **8a** and the second opening **8b** by the capillary force. In the case where the biosensor **101** is configured such that the capillary force does not substantially act on the first opening **8a** or the second opening **8b,** neither the first discharge opening **10ea** nor the second discharge opening **10eb** needs to be provided. In this case, the sample introduced from the introduction opening **10c** flows into the first opening **8a** and the second opening **8b** by the gravity.

The above-described components are stacked, and as a result, as shown in FIG. **1(a)** and FIG. **2(a)****,** a first cell **11a** having the introduction opening **10c,** the first opening **8a** and the first discharge opening **10ea** formed therein and detecting the first substance, and a second cell **11b** having the introduction opening **10c,** the second opening **8b** and the second discharge opening **10eb** formed therein and detecting the second substance, are provided.

The base substrate **2,** the spacer substrate **8** and the top cover substrate **10** are each formed of an insulating material. As the insulating material, for example, any of various resin materials, for example, PET (poly(ethylene terephthalate)) or the like is usable. Instead of such a resin material, an inorganic material such as glass or the like is usable. The electrode layer **4** is formed of a conductive material. The electrode layer **4** is formed by patterning a metal film of, for example, palladium or the like with a laser beam or the like. The base substrate **2,** the electrode layer **4,** the spacer substrate 8 and the top cover substrate **10** are bonded together with, for example, an adhesive.

### <Structure of the measurement device>

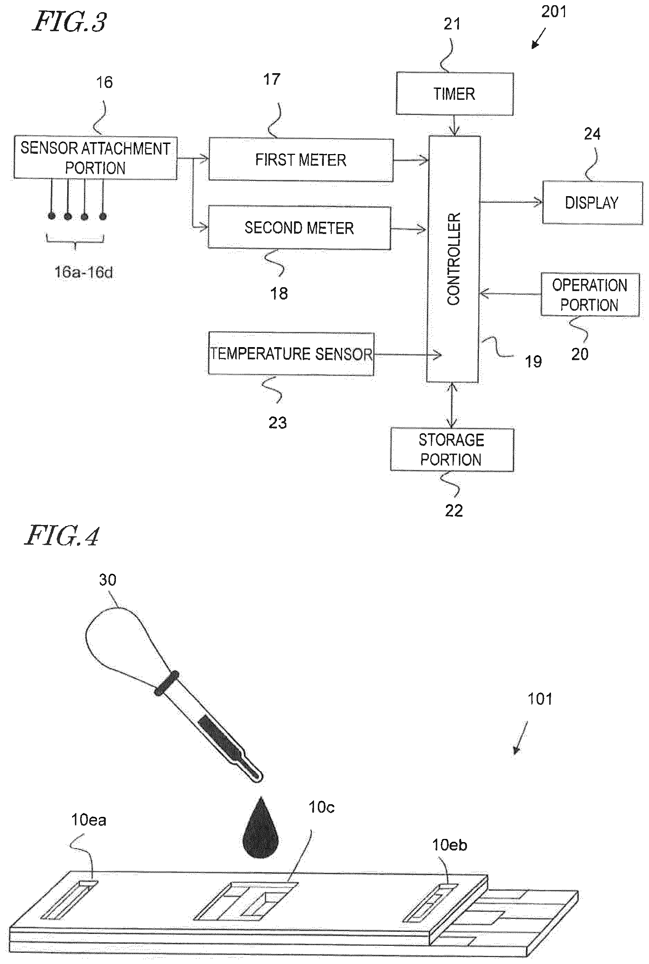

FIG. **3** shows an example of structure of a measurement device **201** measuring the first substance and the second substance contained in the sample by having the biosensor **101** mounted thereon. The measurement device **201** includes a sensor attachment portion **16,** a first meter **17,** a second meter **18,** and a controller **19.** The sensor attachment portion **16** includes connectors **16a** through **16d** in contact with the terminals **4ea** through **4ed** of the biosensor **101** and electrically connected with the first meter **17** and the second meter **18.** The first meter **17** and the second meter **18** apply a predetermined voltage to the first electrode pair **4a** and the second electrode pair **4b** via the connectors **16a** through **16d** and the terminals **4ea** through **4ed** to measure the current values. The measured current values are output to the controller **19.**

The controller **19** determines the concentrations of the first substance and the second substance from the current values (or voltage values) received from the first meter **17** and the second meter **18.** For example, the controller **19** has, stored thereon, a table showing the relationship between the current values and the concentration values or a function of the current values and the concentration values, and calculates the concentrations of the first substance and the second substance based on the table or the function. In this embodiment, the first substance is HbAlc and the second substance is Hb. The ratio of HbAlc with respect to Hb is calculated based on the current values or the concentration values, and the HbAlc value is determined. The measurement device **201** also includes an operation portion **20,** a timer **21,** a storage portion **22,** a temperature sensor **23** and a display **24.** The measurement device **201** may refer to information from the temperature sensor **23** to correct the HbAlc value. The corrected HbAlc value is stored on the storage portion **22** and displayed on the display **24,** together with information on the time supplied by the timer **21.** The controller **19** performs processes such as the measurement, display, storage of the measurement results and the like based on an instruction input by an operator to the operation portion **20.**

The controller **19** of the measurement device **201** includes a computation device such as a microcomputer or the like and a storage portion. The storage portion stores a program defining a procedure of the measurement. The computation device executes the program, and thus the concentrations of the first substance and the second substance are measured. The first meter **17** and the second meter **18** each include a power supply circuit generating a predetermined voltage and a current meter measuring a current value. A measured current value is, for example, converted into a digital signal and processed as described above by the controller **19.** The display **24** may be a liquid crystal display device, an organic EL display device or the like.

### <Measurement of HbA1c by the biosensor 101 and the measurement device 201>

Hereinafter, a specific procedure of measurement performed on HbAlc by use of the biosensor **101** and the measurement device **201** will be described.

### (1) Preprocess on the sample

First, a blood sample obtained from a measurement subject person is preprocessed. Specifically, a hemolytic agent such as a surfactant or the like is added to the obtained blood sample to elute Hb in the erythrocytes in the blood sample.

Next, protease, which is a degradative enzyme, is added to the blood sample containing the eluted Hb to degrade Hb. As a result of the degradation, HbA1c in Hb generates fructosyl valyl histidine. Hereinafter, the preprocessed blood sample will be referred to simply as a "sample".

### (2) Electrochemical reaction caused by the biosensor 101

The biosensor **101** is attached to the measurement device **201.** The sample is absorbed by a dropper **30,** and as shown in FIG. **4****,** is dropped to an area in the vicinity of the introduction opening **10c** of the biosensor **101.** The sample attached to the area in the vicinity of the introduction opening **10c** is pulled through the introduction opening **10c** by the capillary force and is introduced into the first opening **8a** and the second opening **8b** at the same time, namely, in parallel to each other.

Fructosyl valyl histidine in the sample introduced into the first opening **8a** reacts with fructosyl peptide oxidase, which is the first reagent contained in the first reagent layer **6A,** to generate hydrogen peroxide. The conductivity of the sample varies in accordance with the amount of the generated hydrogen peroxide. Therefore, the value of the current flowing between the working electrodes **4aw** and the counter electrodes **4ac** of the first electrode pair **4a** also varies in accordance with the amount of the generated hydrogen peroxide. Since fructosyl valyl histidine is derived from HbA1c, the current value varies in accordance with the amount HbAlc in the sample.

Hb in the sample introduced into the second opening **8b** and potassium ferricyanide, which is the second reagent contained in the second reagent layer **6B,** cause an oxidation-reduction reaction. The movement of charges caused by the oxidation-reduction reaction is detected as a current flowing between the working electrode **4bw** and the counter electrodes **4bc** of the second electrode pair **4b,** and thus the current value in accordance with the amount of Hb is detected.

### (3) Measurement by the measurement device 201

For example, a voltage of 0.1 V to 0.4 V is applied by the measurement device **201** for 5 to 10 seconds between the working electrodes **4aw** and the counter electrodes **4ac** of the first electrode pair **4a,** and the amount of the current flowing between the working electrodes **4aw** and the counter electrodes **4ac** is measured. Similarly, a voltage of 0.1 V to 0.4 V is applied for 5 to 10 seconds between the working electrode **4bw** and the counter electrodes **4bc** of the second electrode pair **4b,** and the amount of the current flowing between the working electrode **4bw** and the counter electrodes **4bc** is measured.

The current values measured by the first meter **17** and the second meter **18** respectively correspond to the amount of HbAlc and the amount of Hb. The measurement device **201** uses these values to find the HbAlc value.

### <Measurement result examples>

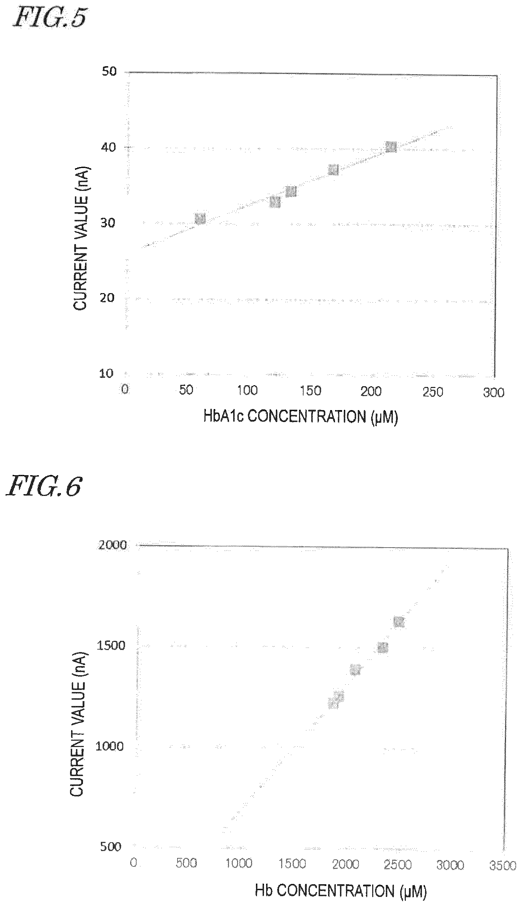

FIG. **5** and FIG. **6** show the results of the measurement performed on samples, the concentrations of HbAlc and Hb of which are known. Five types of samples, the concentrations of HbAlc of which were known in advance, were used to measure the current values by the first meter **17.** In the graph of FIG. **5****,** the concentrations of HbAlc of the samples are represented by the horizontal axis, and the measured current values are represented by the vertical axis. Similarly, five types of samples, the concentrations of Hb of which were known in advance, were used to measure the current values by the second meter **18.** In the graph of FIG. **6****,** the concentrations of Hb of the samples are represented by the horizontal axis, and the measured current values are represented by the vertical axis.

As seen from FIG. **5** and FIG. **6****,** the concentration of HbAlc and the current value have a linear relationship, and the concentration of Hb and the current value also have a linear relationship. It is seen from this that the biosensor **101** of this disclosure is usable to electrochemically measure the concentration of HbAlc and the concentration of Hb and find the HbAlc value with high precision.

### <Effect, etc.>

As described above, in the biosensor of this disclosure, the introduction opening in the top cover substrate overlaps the first opening and the second opening. Therefore, the sample is introduced into the first opening and the second opening in which the electrodes are provided, with no need to provide a flow path including branches for introducing the sample into the reaction chambers. Namely, the biosensor does not include a flow path including branches. Therefore, the area size of the first opening and/or the second opening may be enlarged to increase the amount of the sample used for the measurement, and also the length of the borders between the working electrode(s) and the counter electrodes may be extended. Thus, the detection sensitivity of the biosensor is improved. Since the sample is introduced from the introduction portion, the sample is introduced into the first opening and the second opening provided in the spacer substrate at the same time in parallel to each other. Therefore, the time required to fill the first opening and the second opening with the sample to start the measurement is shortened.

The sample is reacted with the first substance and the second substance respectively in the first opening and the second opening. Therefore, the two types of substances in the sample are detected or quantified by use of the first electrode pair and the second electrode pair. Since the first opening and the second opening are independent from each other, the measurement on either one of the first substance or the second substance does not influence the measurement on the other substance. The two types of substances are detected independently with high precision.

Since the first opening and the second opening are independent from each other, the size of each thereof may be set arbitrarily. The amounts of the sample to be used for the first opening and the second opening are adjusted independently. Therefore, such amounts of the sample are made different in accordance with the concentrations, to be detected, of the substances in the sample, and also in accordance with the detection sensitivity on each of the substances. The measurement is performed with appropriate sensitivity. For example, the area size of the first opening may be made larger than that of the second opening, so that the detection precision on the substance to be detected by use of the first substance is improved. The area size of the first opening may be made larger than that of the second opening to extend the length of the borders between the working electrodes and the counter electrodes of the first electrode pair, so that the detection sensitivity thereof is further improved. The first opening and the second opening are independent from each other. Therefore, in the case where the sample is introduced into the first opening and the second opening by the capillary force, the capillary force acting on each of the first opening and the second opening is independently adjustable.

The top cover substrate includes the introduction portion. Therefore, the amount of the sample to be dropped may be increased to fill the first opening and the second opening with the sample within a short time. This shortens the measurement time. Even if the area size of each of the first electrode pair and the second electrode pair for the measurement is enlarged, the sample of a sufficient amount is put into contact with the electrode pairs within a short time. This improves the detection sensitivity.

The first discharge opening and the second discharge opening are provided respectively for the first opening and the second opening. This allows the sample to be introduced into the first opening and the second opening by use of the capillary force. Therefore, the first opening and the second opening are filled with the sample stably and certainly.

### (EMBODIMENT 2)

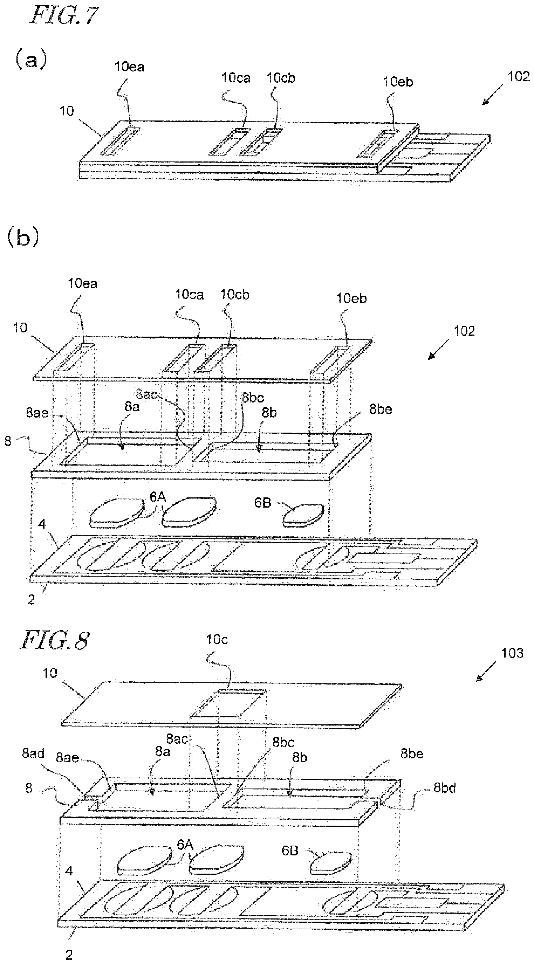

A biosensor in embodiment 2 of this disclosure will be described. FIG. **7(a)** and FIG. **7(b)** are respectively an isometric view and an exploded isometric view of a biosensor **102** in this embodiment. Unlike in the biosensor **101** in embodiment 1, in the biosensor **102,** the top cover substrate **10** includes an introduction portion having a first introduction opening **10ca** and a second introduction opening **10cb** formed therein. The one end **8ac** of the first opening **8a** is located in the first introduction opening **10ca,** and the one end **8bc** of the second opening **8b** is located in the second introduction opening **10cb.** The first introduction opening **10ca** and the second introduction opening **10cb** are independent from each other and are not in communication with each other. Therefore, the area size (size) of each introduction opening is independently adjustable. The area sizes of the first introduction opening **10ca** and the second introduction opening **10cb** respectively overlapping the first opening **8a** and the second opening **8b** may be varied, so that the magnitudes of the capillary forces acting on the first opening **8a** and the second opening **8b** are independently adjusted. Even if the first opening **8a** and the second opening **8b** are different in the area size or the shape, the capillary force acting on each of the first opening **8a** and the second opening **8b** is adjustable to be appropriate. Thus, the sample is introduced into the biosensor **102** more certainly.

The first introduction opening **10ca** and the second introduction opening **10cb** are close to each other. Therefore, for example, the sample, when being dropped to an area between the first introduction opening **10ca** and the second introduction opening **10cb,** is introduced into both of the first introduction opening **10ca** and the second introduction opening **10cb,** and thus is introduced into the first opening **8a** and the second opening **8b** substantially at the same time.

### (EMBODIMENT 3)

A biosensor in embodiment 3 of this disclosure will be described. FIG. **8** is an exploded isometric view of a biosensor **103** in this embodiment. Unlike in the biosensor **101** in embodiment 1, in the biosensor **103,** the spacer substrate **8** has a first discharge opening **8ad** and a second discharge opening **8bd** formed therein. The first discharge opening **8ad** is provided at the other end **8ae** of the first opening **8a** and has an opening at one side surface, in the longitudinal direction, of the spacer substrate **8.** The second discharge opening **8bd** is provided at the other end **8be** of the second opening **8b** and has an opening at the other side surface, in the longitudinal direction, of the spacer substrate **8.**

With this structure, the top cover substrate **10** merely has the introduction opening **10c** formed in a main surface thereof. Therefore, it is less possible that any foreign object enters the first discharge opening **10ea** or the second discharge opening **10eb** or that the operator considers, by mistake, the first discharge opening **10ea** or the second discharge opening **10eb** as an introduction opening and introduces the sample through the first discharge opening **10ea** or the second discharge opening **10eb.**

### (EMBODIMENT 4)

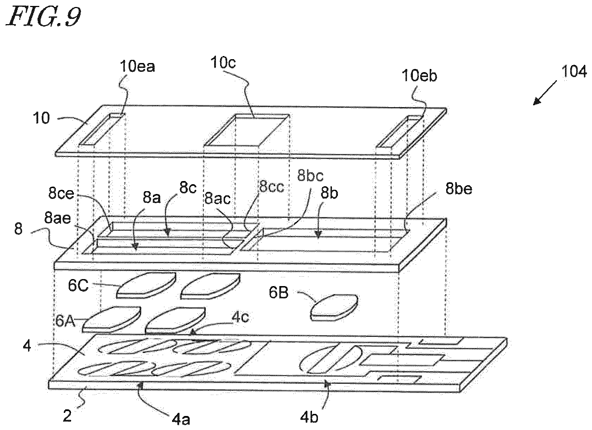

A biosensor in embodiment 4 of this disclosure will be described. FIG. **9** is an exploded isometric view of a biosensor **104** in this embodiment. Unlike in the biosensor **101** in embodiment 1, in the biosensor **104,** the spacer substrate **8** has the first opening **8a,** the second opening **8b** and a third opening **8c** formed therein, the electrode layer **4** further includes a third electrode pair **4c,** and the biosensor **104** includes a third reagent layer **6C.** The first opening **8a** and the third opening **8c** are arranged in a direction perpendicular to the longitudinal direction. The one end **8ac** of the first opening **8a** and one end **8cc** of the third opening **8c** are close to the one end **8bc** of the second opening **8b.** The introduction opening **10c** overlaps the one end **8ac** of the first opening **8a,** the one end **8cc** of the third opening **8c** and the one end **8bc** of the second opening **8b.** The first discharge opening **10ea** overlaps the other end **8ae** of the first opening **8a** and the other end **8ce** of the third opening **8c.** The third electrode pair **4c** is partially exposed to the third opening **8c.** The third reagent layer **6C** is located in the third opening **8c.**

With the biosensor **104,** the sample is introduced from the introduction portion of the top cover substrate **10** and thus is introduced into the first opening **8a,** the second opening **8b** and the third opening **8c** provided in the spacer substrate **8** in parallel to each other. The third reagent layer **6C** may contain a substance reactive with a third substance contained in the sample or a substance derived from the third substance, so that three substances contained in the sample are detected.

### (Other embodiments)

Embodiments 1 through 4 may be combined in an appropriate manner. The number of the openings independently provided in the spacer substrate **8** is not limited to two or three, and may be four or greater. The sample is not limited to blood or a liquid obtained as a result of blood being preprocessed. The first substance and the second substance are not limited to HblAc and Hb. Thus, a biosensor detecting any of various substances is provided.

### INDUSTRIAL APPLICABILITY

A biosensor of this disclosure is preferably usable in the medical and clinical inspection, pharmaceutical manufacturing, or various other industrial fields.

### REFERENCE SIGNS LIST

- **2**: Base substrate
- **2m, 2r, 8m, 8r**: Main surface
- **4**: Electrode layer
- **4a**: First electrode pair
- **4ac, 4bc**: Counter electrode
- **4aw, 4bw**: Working electrode
- **4b**: Second electrode pair
- **4c**: Third electrode pair
- **4da** - **4dd**: Line
- **4ea** - **4ed**: Terminal
- **6A**: First reagent layer
- **6B**: Second reagent layer
- **6C**: Third reagent layer
- **8**: Spacer substrate
- **8a**: First opening
- **8ac, 8bc, 8cc**: One end
- **8ad**: First discharge opening
- **8ae, 8be, 8ce**: The other end
- **8b**: Second opening
- **8bd**: Second discharge opening
- **8c**: Third opening
- **10**: Top cover substrate
- **10c**: Introduction opening
- **10ca**: First introduction opening
- **10cb**: Second introduction opening
- **10ea**: First discharge opening
- **10eb**: Second discharge opening
- **16**: Sensor attachment portion
- **16a** - **16d**: Connector
- **17**: First meter
- **18**: Second meter
- **19**: Controller
- **20**: Operation portion
- **21**: Timer
- **22**: Storage portion
- **23**: Temperature sensor
- **24**: Display
- **30**: Dropper
- **101** - **104**: Biosensor
- **201**: Measurement device
- **ca, cb**: Cut

## Claims

1. A biosensor, comprising:
a base substrate having a main surface;
an electrode layer located on the main surface of the base substrate and including a first electrode pair and a second electrode pair each including a working electrode and a counter electrode;
a spacer substrate located on the electrode layer and having a first opening and a second opening formed therein, the first opening exposing a part of the first electrode pair and the second opening exposing a part of the second electrode pair, the second opening being independent from the first opening;
a first reagent layer containing a first reagent reactive with a sample, the first reagent layer being located in the first opening;
a second reagent layer containing a second reagent reactive with the sample, the second reagent layer being located in the second opening; and
a top cover substrate located on the spacer substrate, the top cover substrate including an introduction portion having at least one introduction opening formed therein, the introduction portion overlapping a part of the first opening in the spacer substrate and a part of the second opening in the spacer substrate.

2. The biosensor according to claim 1, wherein in the spacer substrate, the first opening has an area size larger than an area size of the second opening.

3. The biosensor according to claim 1 or 2, wherein in the electrode layer, a length of a border between the working electrode and the counter electrode adjacent thereto of the first electrode pair exposed to the first opening is longer than a length of a border between the working electrode and the counter electrode adjacent thereto of the second electrode pair exposed to the second opening.

4. The biosensor according to any one of claims 1 through 3, further comprising a first discharge opening and a second discharge opening respectively in communication with the first opening and the second opening in the spacer substrate.

5. The biosensor according to claim 4, wherein the first discharge opening and the second discharge opening are provided in the spacer substrate, each have an opening at a side surface of the spacer substrate, and are respectively connected with the first opening and the second opening.

6. The biosensor according to claim 4, wherein the first discharge opening and the second discharge opening are provided in the top cover substrate and respectively overlap another part of the first opening in the spacer substrate and another part of the second opening in the spacer substrate.

7. The biosensor according to any one of claims 1 through 6, wherein the introduction portion of the top cover substrate has one introduction opening formed therein, and the introduction opening overlaps the part of the first opening in the spacer substrate and the part of the second opening in the spacer substrate.

8. The biosensor according to any one of claims 1 through 6, wherein the introduction portion of the top cover substrate has two introduction openings formed therein, one of the introduction openings overlaps the part of the first opening in the spacer substrate, and the other of the introduction openings overlaps the part of the second opening in the spacer substrate.

9. The biosensor according to any one of claims 1 through 3, wherein:
the base substrate, the spacer substrate and the top cover substrate each have a strip shape that is longer in a first direction than in a second direction perpendicular to the first direction;
the first opening and the second opening in the spacer substrate are located adjacent to each other in the first direction; and
the introduction portion of the top cover substrate has one introduction opening formed therein, and the introduction opening overlaps parts of the first opening and the second opening that are adjacent to each other.

10. The biosensor according to claim 9, wherein:
the top cover substrate further has a first discharge opening and a second discharge opening formed therein;
the first discharge opening and the introduction opening in the introduction portion respectively overlap two ends in the first direction of the first opening in the spacer substrate; and
the second discharge opening and the introduction opening in the introduction portion respectively overlap two ends in the first direction of the second opening in the spacer substrate.

11. The biosensor according to any one of claims 1 through 10, wherein:
the sample contains hemoglobin separated from erythrocytes;
the hemoglobin contains hemoglobin A1c;
the first reagent is specifically reactive with the hemoglobin A1c or a substance derived from the hemoglobin A1c; and
the second reagent is reactive with the hemoglobin.
